# DEMANDE DE BREVET EUROPEEN

(11) **EP 0 880 902 A1**
(43) Date de publication de la demande: **02.12.1998**
(21) Numéro de dépôt: 97201607.5
(22) Date de dépôt: 27.05.1997
(51) Int. Cl.: A23J 1/20, A23C 9/146, C07K 14/47, A61K 7/16

(54) **Procédé de traitement d'une matiére première lactosérique**

(71) Demandeur: Nestlé Produkte AG, 1800 Vevey (CH)
(72) Inventeur: Erdmann, Peter, 3006 Bern (CH); Neumann, Fred, 3612 Steffisburg (CH)
(74) Mandataire: Archambault, Jean

(57) **Abrégé**

Un procédé industriel simple d'échange d'ions consiste à traiter une matière première lactosérique contenant le glycomacropeptide en présence d'une résine anionique faible de manière à obtenir un produit lactosérique amélioré utilisable en alimentation et le dit glycomacropeptide qui est adsorbé sélectivement sur la résine, puis élué de la dite résine.

## Description

L'invention a trait à un procédé de traitement d'une matière première lactosérique contenant le glycomacropeptide ou caséinoglycomacropeptide (ci-après GMP), dans le but d'en séparer le dit GMP.

Le GMP est un macropeptide phosphorylé et partiellement sialylé qui est formé par l'action d'une protéase, par exemple la présure sur la kappa-caséine du lait des mammifères. Il représente environ 20 % en poids des protéines du lactosérum doux obtenu après séparation de la caséine lors de la fabrication de fromages.

On connait un procédé de fabrication de GMP au niveau du laboratoire consistant à traiter une matière première d'origine lactique, telle que par exemple une caséine acide ou un caséinate, hydrolysés par la présure ou encore un lactosérum doux de fromagerie déminéralisé et délactosé, par l'acide trichloracétique de manière à précipiter les protéines, puis à recueillir le sumageant, à le dialyser et enfin à sécher le dialysat. Un tel procédé n'est pas industriel.

Un procédé de production de GMP à l'échelle industrielle, décrit dans EP-A- 0488589 consiste à traiter un produit lactosérique par échange d'ions, à recueillir la fraction n'ayant pas été adsorbée, à la concentrer et à la déminéraliser par ultrafiltration, diafiltration et le cas échéant osmose inverse et à recueillir le GMP.

Un procédé de production d'une fraction protéique de lactosérum est décrit dans UK-A-2188526. Celui-ci consiste à traiter un produit laitier par une résine anionique forte, dans des conditions telles que les protéines et certains peptides de la matière traitée sont adsorbés sur la résine de manière non-sélective sous forme de complexes. De tels complexes sont difficiles à éluer ensuite de la résine. L'éluat se caractérise par la formation d'un gel ferme à pH inférieur à 4,5 et à la température ambiante une fois mis en suspension dans l'eau. La fraction protéique peut être utilisée dans des boissons du genre des "milk-shake" et dans des mousses-desserts.

Le but de l'invention est la séparation sélective du GMP des autres composants des matières lactosériques en une simple opération à l'échelle industrielle avec un rendement élevé.

L'invention concerne donc un procédé de traitement par échange d'ions d'une matière première lactosérique liquide contenant du GMP, dans le but de recueillir d'une part un produit lactosérique utilisable comme source de protéine et d'autre part le GMP sous forme purifiée, caractérisé par le fait qu'il comprend les étapes suivantes:
i) Le cas échéant, ajustement du pH de la matière première lactosérique liquide à une valeur de 1 à 4,3,
ii) Mise en contact du dit liquide avec une résine anionique faible de manière prédominante sous forme alcaline jusqu'à un pH stabilisé de 4,5-5,5,
iii) Séparation de la résine et du produit lactosérique liquide que l'on recueille et
iv) Désorption du GMP de la résine.

Comme matière première lactosérique, on peut utiliser dans le procédé selon l'invention tout produit ou sous-produit contenant le GMP. On peut citer à titre indicatif:
- le lactosérum doux obtenu après séparation de la caséine coagulée par la présure,
- un lactosérum doux ou un tel lactosérum plus ou moins déminéralisé par exemple par électrodialyse, échange d'ions, osmose inverse, électrodéionisation ou une combinaison de ces procédés,
- un concentrat de lactosérum doux,
- un concentrat de lactosérum doux plus ou moins déminéralisé par exemple par électrodialyse, échange d'ions, osmose inverse, électrodéionisation ou une combinaison de ces procédés,
- un concentrat de protéines de lactosérum doux fortement délactosé obtenu par exemple par ultrafiltration, puis diafiltration (ultrafiltration avec lavage),
- les eaux-mères de cristallisation du lactose à partir d'un lactosérum doux,
- un perméat d'ultrafiltration d'un lactosérum doux,
- le produit de l'hydrolyse par une protéase d'une caséine native obtenue par précipitation acide du lait écrémé par un acide minéral ou par acidification biologique, le cas échéant avec addition d'ions calcium,
- le produit de l'hydrolyse d'un caséinate par une protéase.

Une matière lactosérique préférée est un lactosérum doux de fromagerie préconcentré, de préférence à 10-23 % en poids et décationisé ou complétement déionisé.

Une autre matière lactosérique de choix est un concentrat de protéines de lactosérum doux délactosé et décationé.

Ces matières lactosériques peuvent se présenter sous forme liquide ou sous forme de poudre et dans ce dernier cas on les met en dispersion dans l'eau, de préférence déminéralisée en vue de leur traitement ultérieur.

Ces matières lactosériques peuvent provenir de lait de ruminant tel que vache chèvre, brebis ou bufflesse.

Selon un premier mode de réalisation du procédé, on met la matière lactosérique liquide en présence de résine anionique faible dans un réacteur sous agitation modérée à une température < 50° C, de préférence comprise entre 0 et 15° C. L'agitation doit être juste nécessaire à la fluidisation du lit de résine. Celle-ci peut être produite par exemple par un agitateur ou, de préférence par l'introduction d'un courant de fluide, par exemple d'air ou d'azote sous pression par le bas du réacteur.

On peut utiliser toute résine échangeuse d'anions faiblement basique sous forme de gel, macroporeuse ou macroréticulée, de préférence polystyrénique ou polyacrylique, particulièrement à base de copolymère polystyrène divinylbenzène et de préférence macroréticulée pour des raisons de résistance aux chocs osmotiques. Les groupes actifs sont généralement des amines de primaire à tertiaire. Une telle résine doit être de façon prédominante sous forme alcaline (qualifiée ci-après de forme OH⁻) et donc ses sites actifs doivent avoir été régénérés de préférence en grande partie sous cette forme.

Pendant cette mise en contact, les sites actifs de la résine sont échangés contre des molécules de GMP, ce qui produit une augmentation progressive du pH du liquide traité, jusqu'à une valeur finale de 4,5-5,5. La durée de l'opération et les quantités respectives de résine et de liquide traité sont choisies en fonction de la composition du produit de départ et de la quantité de GMP désirée. Cette opération dure de 1 à 10 h, par exemple environ 4 h. Les proportions respectives de résine et de liquide à traiter peuvent varier grandement et sont, en volume de 1:1 à 1:30 et de préférence de 1:1 à 1:10.

Selon un autre mode de réalisation, on peut percoler le liquide dans une colonne remplie de la résine, en recueillir le liquide traité et récupérer le GMP adsorbé sur la résine par élution. On peut pour ce faire procéder en continu ou en semi-continu, par exemple en extrayant la résine saturée de la colonne à contre-courant et en la remplaçant par de la résine fraîchement régénérée.

On peut combiner les modes de réalisation précédents, en réacteur et en colonne, par exemple en utilisant un dispositif mixte dont la partie supérieure est un réacteur muni de moyens d'agitation ou de production d'un lit fluidisé contenant la résine, séparé par une grille ou un filtre d'une partie inférieure constituée d'une colonne où l'on peut réaliser en fin de traitement la récupération de la résine, par exemple par décantation, le soutirage du liquide traité.

Le liquide ainsi traité peut être concentré, par exemple par évaporation, puis séché, par exemple par pulvérisation dans une tour de séchage.

La poudre ainsi obtenue sert avantageusement de matière première protéique dans la préparation des produits infantiles et est remarquable du fait de son profil en acides aminés souhaités, son aminogramme montrant un appauvrissement en thréonine et un enrichissement en acides aminés aromatiques, tels que le tryptophane.

Pour en séparer le GMP, on traite d'abord la résine par lavage, par exemple avec de l'eau déminéralisée, puis le cas échéant avec une solution saline diluée ou une solution acide diluée et on la rince à l'eau déminéralisée. La désorption proprement dite du GMP a lieu avec une solution aqueuse d'acide, de base ou de sel, de préférence avec une solution aqueuse basique, par exemple de NaOH ou KOH, avantageusement de concentration < 8 %, suivie d'un lavage à l'eau déminéralisée. De cette manière, la résine est régénérée par la même occasion. On joint ensuite l'éluat et les eaux de lavage, puis on les déminéralise, par exemple par ultrafiltration ou nanofiltration sur membrane de zone de coupure moyenne environ 3000 dalton et on sèche le rétentat, par exemple par lyophilisation.

Le GMP ainsi obtenu est sensiblement exempt de matière grasse et de lactose et pauvre en protéines de lactosérum.
Il contient de préférence, en poids:
< 1 % de matière grasse,
< 0,2 % de lactose et
< 3 % de protéines vraies de lactosérum.

Le GMP peut être utilisé dans ses applications connues, par exemple pour ses propriétés biologiques dans des compositions pharmaceutiques orales, parentérales ou sous-cutanées comme agent anti-thrombotique, anti-diarrhéique ou anti-bactérien ou de préférence comme agent anti-plaque et anti-carie dans des compositions d'hygiène dentaire, ou encore dans des aliments, par exemple des produits de confiserie pour ses propriétés anti-plaque et anti-carie, pour ses propriétés fonctionnelles d'agent émulsifiant, gélifiant ou moussant ou pour ses propriétés diététiques, par exemple dans des compositions infantiles anti-phénylcétonurie du fait qu'il ne contient pas de phénylalanine.

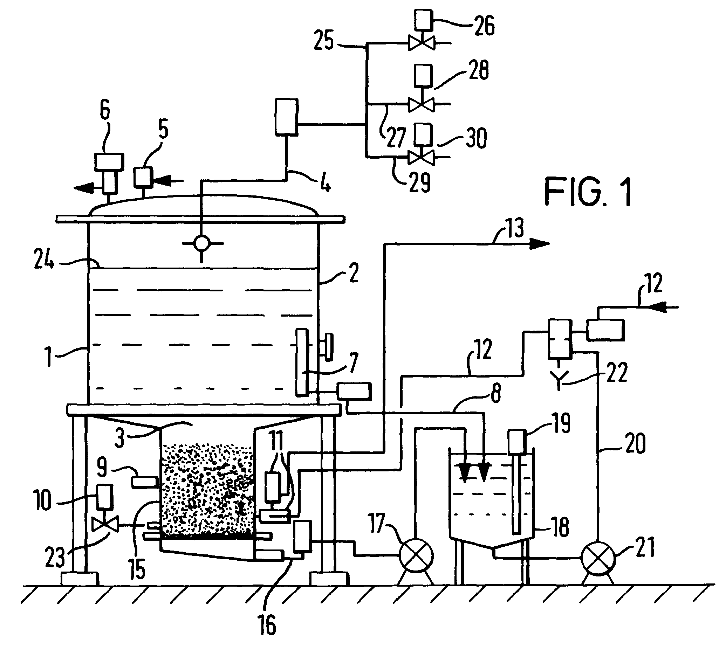

Les exemples ci-après illustrent l'invention, ainsi que la figure 1 du dessin, montrant, de manière schématique et à titre non-limitatif, un dispositif préféré de mise en oeuvre. Dans les exemples, les parties et pourcentages sont pondéraux sauf indication contraire.

### Exemple 1

Pour le traitement, on utilise le réacteur 1 constitué dans sa partie supérieure d'une cuve principale 2 communiquant dans sa partie inférieure avec un compartiment 3 de diamètre plus faible que celui de la cuve 2. La cuve 2 est pourvue d'un conduit d'amenée de liquide de rinçage 4, d'une amenée de gaz sous pression 5, d'une soupape de sécurité 6 permettant de réguler la pression de gaz dans le réacteur 1. A un niveau voisin de sa base la cuve 2 est pourvue d'une crépine 7 et d'un conduit de soutirage de liquide 8.

Au niveau du compartiment 3, le réacteur est muni d'un pH-mètre 9, d'une amenée de gaz 10 et communique par une vanne à trois voies 11 avec un conduit 12 d'amenée de liquide à traiter et un conduit d'évacuation 13 pour le liquide traité. A la base le compartiment 3 est pourvu d'une grille ou d'une plaque perforée 14 dont le rôle est de recueillir les billes de résine 15. En dessous de la grille 14, un conduit 16 de soutirage amène le liquide par l'intermédiaire de la pompe 17 vers la cuve tampon 18 munie d'un dispositif de contrôle de niveau 19 et delà vers le conduit 20 par l'intermédiaire de la pompe 21. Le conduit 20 est relié soit au conduit 12, soit au trop-plein d'évacuation 22.

On disperse un concentrat de protéines de lactosérum doux bovin, traité de manière classique par électrodialyse et décationé sur résine cationique forte, dans de l'eau déionisée de sorte que la solution ait une teneur en matière sèche de 7,5 %.

Le concentrat a la composition ci-après:

| | % |
|---|---|
| Protéines (GMP inclus) | 76 |
| Lactose | 4,8 |
| Cendres | 2,5 |
| Lipides | 8 |
| Eau | complément à 100 |

Par le conduit 12, on transfère 110 kg de la dispersion, de pH initial 4,25, à la température de 12° C dans le réacteur 1 par la base duquel on introduit de l'air en barbottage au niveau du compartiment 3, par l'amenée 10 par l'intermédiaire d'une vanne de non retour 23, de manière à créer un lit fluidisé de billes de résine 15 comprenant 23 kg de résine anionique faible (IMAC HP 661^{(R)}, Rohm & Haas, régénérée sous forme OH⁻). Les billes de résine 15 sont agitées pendant 4 h au contact de la dispersion du fait de la turbulence créée par la fluidisation. On contrôle constamment le pH au moyen du pH-mètre 9. La stabilisation du pH à 5,08 indique la fin de la réaction. On coupe alors l'arrivée d'air en 10 et on introduit de l'air par le haut du réacteur en 5 au dessus du niveau 24 de liquide, ce qui a pour effet de pousser le liquide et de décanter les billes de résine dans la partie inférieure 3 du réacteur 2 où elles sont retenues par la grille 14. On soutire le liquide traité par gravité par le conduit 8 et par le conduit 16 par l'intermédiaire de la pompe 17 vers la cuve tampon 18 et on l'évacue par le conduit 20 au moyen de la pompe 21 et delà vers la sortie par les conduits 12 et 13.

Après concentration du liquide à 28 % de matières sèches par évaporation, on sèche le concentrat par pulvérisation dans une tour de séchage (ces opérations n'étant pas représentées).

Une analyse du concentrat par chromatographie liquide à haute performance (HPLC) montre que la réaction a enlevé 91 % du GMP de départ. Par ailleurs, la poudre contient 95 % des protéines de lactosérum de départ.

Pour recueillir le GMP, on lave le réacteur et la résine avec de l'eau déionisée depuis le conduit 25 et la vanne 26, puis le conduit 4 à travers le réacteur jusqu'à la sortie par les conduits 12 et 13. Par le même circuit, on élue le GMP avec deux fois 40 l de solution aqueuse à 2 % de NaOH distribuée par le conduit 27 et la vanne 28 et on rince avec 30 l d'eau déionisée. Après avoir joint les volumes d'éluat et de rinçage, on concentre l'ensemble à un volume de 25 l par ultrafiltration ou nanofiltration avec une membrane de coupure nominale de 3.000 dalton, puis l'on sèche le rétentat par lyophilisation (ces opérations n'étant pas représentées) et l'on obtient 750 g de GMP, ce qui correspond à un rendement de 82 % par rapport au GMP de départ.

Périodiquement, la résine subit une régénération acide après la régénération alcaline une fois que l'on a traité l'équivalent de 10 volumes de lit de résine. Pour ce faire, après l'élution du GMP par la solution alcaline tel que décrit précédemment, on amène une solution aqueuse concentrée de HCl par le conduit 29 et la vanne 30, respectivement 25 pour l'eau. La résine est ensuite mise sous forme OH⁻ par passage d'une solution aqueuse concentrée de NaOH depuis les conduits 27, respectivement 25 pour l'eau, puis 4, et sort ensuite du réacteur 1 par le conduit 16, est repris par la pompe 17 vers la cuve tampon 18, puis par la pompe 21 et évacué par le conduit 20 et le trop-plein 22 vers le traitement des effluents. A la suite de cette opération, la résine est prête pour un autre cycle de traitement.

### Exemple 2

On utilise un lactosérum doux bovin, préalablement concentré à 17 % de matière sèche, puis déminéralisé par électrodialyse, décationé sur colonne de résine cationique forte, désanioné sur colonne de résine anionique faible et séché par pulvérisation en tour de séchage, de composition indiquée ci-après:

| | % |
|---|---|
| Protéines (GMP inclus) | 11,7 |
| Lactose | 81,7 |
| Cendres | 1 |
| Lipides | 1 |
| Eau | complément à 100 |

On solubilise cette poudre de lactosérum déminéralisée dans de l'eau déionisée et la solution a un pH initial de 3,8. Dans l'installation précédente, on traite 392 kg de cette solution à la température de 8° C en l'agitant dans le réacteur en présence de 23 kg de résine anionique faible (IMAC HP 661^{(R)} , Rohm & Haas, régénérée sous forme OH⁻) pendant 4 h. La stabilisation du pH à 4,89 indique la fin de la réaction. On soutire alors le liquide et on recueille la résine comme précédemment. Après concentration du liquide à 28 % de matières sèches par évaporation, on sèche le concentrat par pulvérisation dans une tour de séchage.

Une analyse du concentrat par HPLC montre que la réaction a enlevé 89 % du GMP de départ. Par ailleurs, la poudre contient 9,1 % de protéines de lactosérum, ce qui correspond à un rendement de 90 % des protéines de lactosérum.

Pour recueillir le GMP, on lave la résine successivement avec de l'eau déionisée, avec 30 l d'une solution aqueuse à 0,5 % de HCl et avec 30 l d'eau déionisée, puis on élue le GMP avec deux fois 40 l de solution aqueuse à 2 % de NaOH et on rince avec 30 l d'eau désionisée. Après avoir joint les volumes d'éluat et de rinçage, on concentre l'ensemble à un volume de 25 l par ultrafiltration avec une membrane de coupure nominale de 3.000 dalton, puis l'on sèche le rétentat par lyophilisation et l'on obtient 900 g de GMP, ce qui correspond à un rendement de 80 % par rapport au GMP de départ.

### Exemple 3

On part d'un lactosérum doux, préconcentré à 18 % de matière sèche, décationé par traitement sur une colonne de résine cationique forte, dont le pH initial est 1,09.

Dans l'installation précédente, on traite 70 kg de ce lactosérum à la température de 25° C en l'agitant dans le réacteur en présence de 14 kg de résine anionique faible (IRA 96^{(R)} , Rohm & Haas, régénérée sous forme OH⁻) pendant 4 h. L'agitation se fait par création d'un lit fluidisé de billes de résine avec barbottage d'azote. La stabilisation du pH à 4,79 indique la fin de la réaction. On sépare alors le liquide de la résine comme précédemment. Après concentration du liquide à 28 % de matières sèches par évaporation, on sèche le concentrat par pulvérisation dans une tour de séchage.

Une analyse de la poudre par HPLC montre que la réaction a enlevé 85 % du GMP de départ. Par ailleurs, la poudre contient 9,2 % des protéines de lactosérum, ce qui correspond à un rendement de 90 % des protéines de lactosérum.

Pour recueillir le GMP, on lave la résine successivement avec de l'eau déionisée, avec 50 l d'une solution aqueuse à 0,05 % de NaCl et deux fois 50 l d'eau déionisée, puis on élue le GMP avec deux fois 25 l de solution aqueuse à 2 % de NaOH et on rince avec 10 l d'eau déionisée. Après avoir joint les volumes d'éluat et de rinçage, on concentre l'ensemble à un volume de 25 l par ultrafiltration avec une membrane de coupure nominale de 3.000 dalton, puis l'on sèche le rétentat par lyophilisation et l'on obtient 175 g de GMP, ce qui correspond à un rendement de 80 % par rapport au GMP de départ.

### Exemple 4

On percole 3,5 l de lactosérum doux préconcentré à 20 % de matière sèche, décationé sur colonne de résine cationique forte et de pH 1,09 à travers une colonne contenant 450 ml de résine anionique faible (IMAC HP 661^{(R)} , Rohm & Haas), à raison de 2 volumes de lit/h.

On recueille l'équivalent de 4 volumes de lit, constituant 4 fractions égales de pH allant de 6 à 3 et dont la quantité de GMP enlevé va de 50 à 9 % (évalué par HPLC). Après réunion des 4 fractions, on obtient une solution de pH 4,5 dans laquelle 25 % du GPM a été enlevé (par rapport à la matière lactosérique de départ).

Pour recueillir le GMP, on procède comme à l'exemple 1 et on obtient des résultats équivalents quant à la pureté du GMP.

## Revendications

1. Procédé de traitement par échange d'ions d'une matière première lactosérique liquide contenant du GMP, dans le but de recueillir d'une part un produit lactosérique utilisable comme source de protéine et d'autre part le GMP sous forme purifiée, caractérisé par le fait qu'il comprend les étapes suivantes:
i) Le cas échéant, ajustement du pH de la matière première lactosérique liquide à une valeur de 1 à 4,3,
ii) Mise en contact du dit liquide avec une résine anionique faible de manière prédominante sous forme alcalinejusqu'à un pH stabilisé de 4,5-5,5,
iii) Séparation de la résine et du produit lactosérique liquide que l'on recueille et
iv) Désorption du GMP de la résine.

2. Procédé selon la revendication 1, caractérisé par le fait que la matière lactosérique est un lactosérum doux de fromagerie préconcentré, de préférence à 10-23 % en poids et décationé ou complétement déionisé.

3. Procédé selon la revendication 1, caractérisé par le fait que la matière première lactosérique est un concentrat de protéines de lactosérum doux délactosé et décationé.

4. Procédé selon la revendication 1, caractérisé par le fait que l'on met la matière première lactosérique liquide en présence de résine anionique faible de manière prédominante sous forme alcaline dans un réacteur sous agitation modérée à une température < 50° C, de préférence comprise entre 0 et 15° C, pendant une à plusieurs heures, ce qui produit une augmentation progressive du pH du liquide traité, jusqu'à une valeur finale de 4,5-5,5, puis que l'on sépare le liquide de la résine par filtration ou centrifugation.

5. Procédé selon la revendication 4, caractérisé par le fait que l'on utilise toute résine échangeuse d'anions faiblement basique sous forme de gel, macroporeuse ou macroréticulée.

6. Procédé selon la revendication 4, caractérisé par le fait que l'on concentre le liquide ainsi traité, notamment par évaporation, puis qu'on le sèche, notamment par pulvérisation dans une tour de séchage.

7. Procédé selon la revendication 1, caractérisé par le fait que, pour en séparer le GMP sous forme purifiée, on traite d'abord la résine par lavage, puis on désorbe le GMP avec une solution aqueuse acide, basique ou saline, notamment de NaOH ou KOH, de concentration < 8 %, on la rince à l'eau déminéralisée, on joint ensuite l'éluat et les eaux de rinçage, puis on les déminéralise, notamment par ultrafiltration ou nanofiltration sur membrane de zone de coupure moyenne environ 3000 dalton et que l'on sèche le rétentat, notamment par lyophilisation.

8. Utilisation du produit lactosérique obtenu par le procédé selon l'une des revendications 1 à 6 comme matière première protéique dans la préparation des produits infantiles et diététiques conduisant à un profil en acides aminés caractérisé par un appauvrissement en thréonine et un enrichissement en acides aminés aromatiques, notamment en tryptophane.

9. Utilisation du GMP purifié obtenu par le procédé selon la revendication 7 dans des compositions pharmaceutiques comme agent anti-thrombotique, anti-diarrhéique ou anti-bactérien ou encore dans des aliments, notamment des produits de confiserie pour ses propriétés anti-plaque et anti-carie, pour ses propriétés fonctionnelles d'agent émulsifiant, gélifiant ou moussant ou pour ses propriétés diététiques, notamment dans des compositions infantiles.

10. Utilisation du GMP purifié obtenu par le procédé selon la revendication 7 comme agent anti-plaque et anti-carie dans des compositions d'hygiène dentaire.
